# EUROPÄISCHE PATENTANMELDUNG

(11) **EP 1 281 392 A1**
(43) Veröffentlichungstag der Anmeldung: **05.02.2003**
(21) Anmeldenummer: 01402096.0
(22) Anmeldetag: 02.08.2001
(51) Int. Cl.: A61K 7/48, A61K 7/06, A61K 35/78

(54) **Kosmetische und/oder pharmaceutische Zubereitungen enthaltend Pflanzenextrakte**

(71) Anmelder: Cognis France S.A., 31360 Saint-Martory (FR)
(72) Erfinder: Pauly, Gilles, 54000 Nancy (FR); Moser, Philippe, 54270 Essey-Les-Nancy (FR); Danoux, Louis, 54420 Saulxures-Les-Nancy (FR)
(74) Vertreter: Cabinet HERRBURGER

(57) **Zusammenfassung**

Vorgeschlagen werden kosmetische und/oder pharmazeutische Zubereitungen, enthaltend einen Extrakt der Pflanze Cajanus cajan. Die Erfindung betrifft weiterhin die vielfältige Verwendung von Extrakten aus Cajanus cajan in Pflegemitteln für Haut und/oder Haare, beispielsweise in Sonnenschutzmitteln, als anti-inflammatorische Mittel, als Antioxidantien bzw. Radikalfänger, als Mittel gegen die Hautalterung, als Proteaseinhibitoren, als Anti-Glykosylierungsmittel und als Haarpflegemittel zur Verbesserung der Kämmbarkeit. Desweiteren betrifft die Erfindung ein Verfahren zur Herstellung eines Extraktes aus Cajanus cajan.

## Beschreibung

### Gebiet der Erfindung

Die Erfindung befindet sich auf dem Gebiet der Kosmetik und betrifft Zubereitungen enthaltend spezielle Pflanzenextrakte sowie die Verwendung dieser Pflanzenextrakte in kosmetischen und/oder pharmazeutischen Zubereitungen beispielsweise für die Hautbehandlung.

### Stand der Technik

Die Aufgabe der Haut als ein den Organismus umhüllendes Organ besteht in abdichtenden und vermittelnden Funktionen gegenüber der Umwelt. Verschiedene biochemische und biophysikalische Systeme dienen der Aufrechterhaltung der Integrität dieses exponierten Organs. Beispielsweise schützt ein Immunsystem die Haut vor Schäden durch pathogene Mikroorganismen, das Melanin bildende System regelt die Pigmentierung und bewahrt die Haut vor Strahlenschäden, ein Lipidsystem produziert Lipidmizellen, die den transdermalen Wasserverlust eindämmen, und eine geregelte Keratinsynthese liefert die mechanisch widerstandsfähige Hornschicht. Den genannten Systemen liegen komplexe chemische Prozesse zu grunde, deren Ablauf unter anderem durch Enzyme in Gang gehalten und durch Enzyminhibitoren geregelt wird. Bereits eine geringfügige Hemmung oder Enthemmung dieser biochemi schen Systeme äussert sich in spürbaren Veränderungen der Haut. Der sichtbare und fühlbare Zustand der Haut gilt jedoch als Massstab für Schönheit, Gesundheit und Jugend; ihn zu erhalten ist ein generelles Ziel pflegender Kosmetik.

Die menschliche Haut reagiert in der Regel auf exogene, d.h. externe Stressfaktoren, wie UV-Strahlung, Ozon oder andere in der Luft vorhandene schädliche Substanzen (Umweltverschmutzung) mit leichten oder schwereren Irritationen. Insbesondere wird die Haut durch die in Irritationsreaktionen freigesetzten Sauerstoffradikale und nichtspezifischen Proteinasen geschädigt. Dies kann beispielsweise das Aussehen oder die Elastizität oder die Barrierefunktionen der Haut negativ beeinflussen. So können bei entzündlichen Prozessen und Immunreaktionen im Überschuss mobilisierte körpereigene Proteasen, wie zum Beispiel Tryptasen, Elastasen, Collagenasen und Cathepsine, die Haut und im besonderen deren Strukturproteine wie Collagen und Elastin angreifen.

Pflanzenextrakte werden seit vielen Jahren in den unterschiedlichsten Kulturen für medizinische aber auch für kosmetische Zwecke genutzt. Es werden immer wieder neue Pflanzen extrahiert und die Extrakte auf ihre kosmetischen Wirkungen hin untersucht um weitere Pflanzen mit neuem oder verändertem Wirkspektrum zu finden. Viele Pflanzen, deren Nutzen man noch nicht kannte, und die als exotisch und unbedeutend galten, finden heute breite Anwendung unter anderem in der Kosmetik.

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Von besonderem Interesse sind Stoffe, die sowohl die technischen Eigenschaften des kosmetischen Produktes, wie Lagerstabilität, Lichtstabilität und Formulierbarkeit positiv beeinflussen, als auch gleichzeitig Wirkstoffe darstellen, die für Haut und/oder Haare beispielsweise pflegende, irrtitationshemmende, entzündungshemmende und/oder lichtschutzwirkende Eigenschaften vermitteln. Hierbei sind zusätzlich eine gute Hautverträglichkeit und besonders der Einsatz natürlicher Produkte beim Kunden gefragt.

Darüber hinaus besteht ein generelles Bedürfnis, kosmetische und pharmazeutische Zubereitungen bereitzustellen, die auf Grund ihrer speziellen Zusammensetzung qualitativ gute technische Eigenschaften aufweisen und die sich außerdem durch zusätzliche Eigenschaften für Haut und Haar auszeichnen.

Besonders in den Grenzgebieten zwischen Kosmetik und Pharmazie wächst ein großes Interesse an Pflegestoffen, die pharmazeutische Wirksamkeiten zeigen mit sehr geringen Nebenwirkungen. Werden diese Pflegestoffe in kosmetischen Mitteln angeboten so bietet es dem Verbraucher die Möglichkeit bequem und ohne großen Aufwand Mangelerscheinungen zu beheben oder zu verhindern.

### Beschreibung der Erfindung

Die Aufgabe der vorliegenden Patentanmeldung hat darin bestanden, kosmetische und/oder pharmazeutische Zubereitungen zur Verfügung zu stellen, welche den Anforderungen für kosmetische Formulierungen wie Lagerstabilität und Hautverträglichkeit gerecht werden und neben pflegenden Eigenschaften vor allem verbesserte schützende Eigenschaften für menschliche Haut und/oder Haare beispielsweise gegen UV-Strahlung und anderen Umwelteinflüssen besitzen und gleichzeitig vorbeugende und heilende Wirkung bei Alterserscheinungen der Haut zeigen und antiinflammatorisch einsetzbar sind,

Eine weitere Aufgabe der vorliegenden Patentanmeldung hat darin bestanden, Zubereitungen zur Verfügung zu stellen, die Wirkstoffe aus nachwachsenden Rohstoffen enthalten und gleichzeitig vielseitig als Pflegemittel in der Kosmetik sowohl in der Hautkosmetik, als auch in der Haarpflege einsetzbar sind.

Gegenstand der Erfindung sind kosmetische und/oder pharamzeutische Zubereitungen enthaltend einen Extrakte der Pflanze Cajanus cajan. Bevorzugt sind kosmetische und/oder dermopharmazeutische Zubereitungen.

Unter dem Begriff Pflanze sind im Sinne der vorliegenden Anmeldung sowohl ganze Pflanzen als auch Pflanzenteile (Äste, Blätter, Blüten, Fruchtkörper, Fruchthüllen, Wurzel) sowie deren Gemische zu verstehen. Bevorzugt ist die Extraktion von Blättern.
Die Begriffe Zubereitung, Mittel und Pflegemittel werden im Sinne der Erfindung synonym verwendet.

### Cajanus cajan

Die erfindungsgemäß einzusetzenden Extrakte werden aus der Pflanze Cajanus cajan gewonnen. Diese Pflanze ist auch unter den Synonyma Cajanus indicus, Cytisus cajan, Straucherbse, Taubenerbse, Erbsenbohne und Pigeon pea bekannt. Bei dieser Pflanze handelt es sich um 0,5 bis 4 m hohe, breit verästelte Sträucher oder Büsche, mit einem bis zu 2 m langem dünnen Wurzelwerk die auch als Pfahlwurzeln bezeichnet werden, Die Stämme sind bis zu 15 cm im Durchmesser. Die Blätter erscheinen wechselseitig als dreiblättrige, drüsige, eliptische Blätter mit einer Größe von 13-13,7 cm x 1,3 - 5,7 cm. Sie bringt gelbe oder rotgelbe Blüten in aufrechten lockeren Trauben hervor. Ihre dicht behaarten charakteristischen, oft sichelförmigen Hülsen umschliessen bis zu 8 erbsengroße Samen von weisser bis creme-brauner Farbe. Teilweise werden die Samen auch purpur, gefleckt oder schwarz, Diese Samen werden unreif wie grüne Erbsen zum Teil mit den Hülsen verzehrt oder reif zu Mehl vermahlen. Die Pflanze kann bis zu 5 jahre alt werden, sie wird jedoch in den meissten Fällen nur als einjährige Nutzpflanze angebaut. Die Pflanze stammt ursprünglich aus Indien, ist jedoch mittlerweile in Süd-Ost Asien und in Süd-Afrika weit verbreitet. Sie wird hauptsächlich angebaut wegen der Samen, die zum Verzehr geeignet sind. Des weiteren dient sie als Gründüngungs- und Heckenpflanze an Reisfeldern. Durch das extensive Wurzelwerk verbessert sie die Bodenbeschaffenheit und dient als Stickstoff fixierende Pflanze. Sie dient als Wirt für Seidenwürmer und "lac" Insekten.

In der traditionellen Medizin wird die Pflanze und besonders die Blätter bereits als Diuretikum verwendet. Sie wird eingesetzt gegen die unterschiedlichsten Hauterkrankungen. Die Blätter und unreifen Früchte werden als Umschläge zur Verbesserung der Milchabsonderung eingesetzt.

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf **Hagers Handbuch der Pharmazeutischen Praxis,** (5. Auflage, Bd. 2, S. 1026-1030, Springer Verlag, Berlin-Heidelberg-New-York 1991) verwiesen. Als Ausgangsmaterial können frische Pflanzen oder Pflanzenteile eingesetzt werden, üblicherweise wird jedoch von Pflanzen und/oder Pflanzenteilen ausgegangen, die vor der Extraktion mechanisch zerkleinert werden können. Besonders bevorzugt ist die Extraktion der Blätter. Hierbei eignen sich alle dem Fachmann bekannten Zerkleinerungsmethoden, als Beispiel sei die Zerstoßung mit einem Mörser genannt.

Als Lösungsmittel für die Durchführung der Extraktionen können vorzugsweise organische Lösungsmittel, Wasser (destilliert oder nicht destilliert, vorzugsweise heißes Wasser einer Temperatur von über 80 °C) oder Gemische aus organischen Lösungsmitteln und Wasser, insbesondere niedermolekulare Alkohole bevorzugt Methanol, Ethanol und Propanol, Ester, Kohlenwasserstoffe, Ketone oder halogenhaltige Kohlenwasserstoffe mit mehr oder weniger hohen Wassergehalten, verwendet werden. Besonders bevorzugt ist die Extraktion mit Wasser, Methanol, Ethanol, Pentan, Hexan, Heptan, Aceton, Propylenglycolen, Polyethylenglycolen Ethylacetat, Dichlormethan, Trichlormethan sowie Mischungen hieraus, insbesondere bevorzugt sind Wasser, Methanol, Ethanol sowie Mischungen hieraus. Die Extraktion erfolgt in der Regel bei 20 bis 100 °C, bevorzugt bei 30 bis 90 °C, insbesondere bei 50 bis 90 °C. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Bevorzugt ist eine einstündige Extraktion unter Rühren. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion liegen im Bereich von 3 bis 20, insbesondere 10 bis 17 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Besonders bevorzugt werden die Extrakte anschließend einer Sprüh- oder Gefriertrocknung unterworfen.

Kosmetische und/oder pharmazeutische Zubereitungen auf Basis der Pflanze Cajanus cajan zeigen überraschend gute pflegende und schützende Eigenschaften für Haut und Haar, gegen Stress und gegen Umwelteinflüsse sowie gleichzeitig gute Hautverträglichkeit. Die so erhaltenen Zubereitungen zeichnen sich weiterhin durch eine hohe antioxidative Kapazität aus, die zum einen die Haut vor entzündlichen Reaktionen sowie vor oxidativ bedingten Hautalterungsvorgängen schützt, zum anderen werden gleichzeitig die kosmetischen Mittel vor oxidativem Abbau (Verderb) geschützt. Darüber hinaus sind die so erhaltenen Produkte geeignet, der Schädigung von menschlichen Fibroblasten und Keratinocyten durch UV-Strahlung entgegen zu wirken und können demnach als Sonnenschutzmittel in der Kosmetik eingesetzt werden. Sie zeigen Proteinase-inhibierende Eigenschaften und wirken einer nicht-enzymatischen Glykosylierung entgegen; beide Effekte schützen ebenfalls gegen die Hautalterung.

Die erfindungsgemäßen Extrakte enthalten einen Wirkstoffgehalt in den Extrakten von 5 bis 100 Gew.%, vorzugsweise 10 bis 95 Gew.%, insbesondere 20 bis 80 Gew.%. Der Wirkstoffgehalt im Sinne der Erfindung bezeichnet die Summe aller im Extrakt vorhandenen Wirkstoffe bezogen auf das Trockengewicht des Extraktes.

Wirkstoff im Sinne der Erfindung bezieht sich auf die im Extrakt enthaltenen Inhaltstoffe auch wenn deren Gehalt und Identität mit herkömmlichen, dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

Die Einsatzmenge der Pflanzenextrakte in den genannten Zubereitungen richtet sich nach der Konzentration der einzelnen Inhaltsstoffe und nach der Art der Anwendung der Extrakte. In der Regel werden 0,001 bis 25 Gew.-%, insbesondere 0,03 bis 10 Gew.-%, 0,01 bis 8 Gew.-%, bevorzugt 0,1 bis 5 Gew.-% und besonders bevorzugt 1 bis 3 Gew.-% des Pflanzenextraktes - berechnet als Trockengewicht bezogen auf die Endzubereitung der kosmetischen und/oder pharmazeutischen Zubereitungen eingesetzt, mit der Maßgabe, dass sich die Mengenangaben mit weiteren Hilfs- und Zusatzstoffen und mit Wasser zu 100 Gew.-% addieren.
Der Gesamtanteil der Hilfs- und Zusatzstoffe kann 1 bis 50, vorzugsweise 5 bis 40 Gew.-% - bezogen auf die Endzubereitung der kosmetischen und/oder pharmazeutischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

### Extrakte:

Die erfindungsgemäßen Extrakte der Pflanze Cajanus cajan enthalten in der Regel Inhaltsstoffe aus der Gruppe bestehend aus Tanninen, Phytosterolen, Proteinen, Kohlenhydraten, Phenolsäuren und Triterpenen. Diese sind je nach gewähltem Ausgangsmaterial und nach gewählter Extraktionsmethode unterschiedlich zusammengesetzt.

Unter **Tanninen** sind im Sinne der vorliegenden Erfindung solche zu verstehen, die sich aus der Pflanze Cajanus cajan isolieren lassen. Im Besonderen handelt es sich um Polyphenole, die aufgrund ihrer Abstammung von Gallussäure auch als Gallotannine bezeichnet werden. Sie stellen Gemische von Stoffen vom Typ der Pentadigalloylglucose (C76H52O46, MR 1701,22) dar. Es handelt sich weiterhin um Stoffe, die durch oxidative Kupplung der Galloyl-Reste in 1,2,3,4,6-Pentagalloyl-D-glucose entstehen, sowie um deren Folgeprodukte.

Unter **Phytosterolen** sind im Sinne der Erfindung Sterole zu verstehen, die sich aus der Pflanze Cajanus cajan isolieren lassen. Sie besitzen eine Doppelbindung an C-22 und C1- oder C2-Substituenten an C-24. Besonders bevorzugt sind Ergosterol, Stigmasterol, Sitosterol, insbesondere β-Sitosterol.

Unter **Proteinen** sind im Sinne der Erfindung alle Proteine zu verstehen, die sich aus der Pflanze Cajanusn cajan isolieren lassen. Ihr Anteil am Trockengewicht des Extraktes liegt zwischen 15 und 25 %, insbesondere 15 bis 20 %. Proteine sind ein Bestandteil des Pflanzenplasmas und finden sich daher in allen Pflanzenteilen, hauptsächlich jedoch in den Früchten.

Im Sinne der Erfindung sind unter **Kohlenhydrate** solche zu verstehen, die sich aus der Pflanze Cajanus cajan isolieren lassen. Bevorzugt darunter zu verstehen sind Cellulose, Glucan, Inulin, Agar-Agar, Carrageenan und Alginsäure. Neben Cellulose findet sich ebenfalls Lignin im Pflanzenextrakt.

Im Sinne der Erfindung sind unter Phenolsäuren solche zu verstehen, die sich aus der Pflanze Cajanuns cajan isolieren lassen. Sie kommen in freier Form vor oder als Ester oder Glycoside. Bevorzugt darunter zu verstehen sind p-Hydroxybenzoesäure und o-Hydroxybenzoesäure, Protocatechuinsäure, Vanillinsäure, Kaffeesäure, p-Coumarinsäure, Ferulasäure oder Salicylsäure.

Unter **Triterpenen** sind im Sinne der Erfindung solche Triterpene zu verstehen, die sich aus der Pflanze Cajanus cajan isolieren lassen. Die erfindungsgemäßen Triterpene lassen sich formal als Polymerisationsprodukte des Kohlenwasserstoffs Isopren auffassen. Aus drei Isopren-Resten bilden sich die Triterpene (C30). Aus unterschiedlichen Faltungsmöglichkeiten der drei Isopren-Reste lassen sich verschiedene polycyclische Ring-Systeme für die möglichen Triterpene ableiten. Die Cyclisierung liefert bevorzugt 6-Ringe, daneben bei den meisten tetra- (z. B. Cucurbitacine) und einigen pentacyclischen (z. B. Lupane) Triterpenen noch 5-Ringe. Da die 6-Ringe in der Sessel- und Wannen-Form vorliegen, die 5-Ringe eben oder gewinkelt sein können, sind viele verschiedene Gerüste möglich.

Die vorliegenden Erfindung schließt die Erkenntnis ein, dass durch das Zusammenwirken der Inhaltsstoffe der Pflanzenextrakte, insbesondere der oben genannten, besonders wirkungsvolle kosmetische Mittel erhalten werden.

Weitere Gegenstände der Erfindung betreffen die vielfältige Verwendung der Pflanzenextrakte aus Cajanus cajan beispielsweise
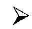 in Pflegemittel für Haut und/oder Haare
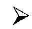 in Pflegemitteln mit lindernder, wohltuender und irritationshemmender Wirkung auf die Haut; insbesondere für sensitive Haut;
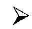 in Sonnenschutzmitteln, insbesondere in Mitteln gegen die Schädigung von menschlichen Hautzellen durch UV-Strahlung, insbesondere von Fibroblasten und/oder Keratinocyten durch UVA-Strahlung und/oder UVB-Strahlung;
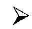 als anti-inflammatorische Additive;
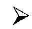 als Antioxidantien bzw. als Radikalfänger;
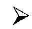 als Mittel gegen Rosacea;
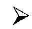 als Mittel gegen hormonell und/oder bakteriell bedingte Hautveränderungen, insbesondere gegen Akne
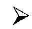 in Pflegemitteln gegen die Hautalterung zur vorbeugenden oder heilenden Behandlung von Alterserscheinungen der Haut;
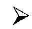 als Protease-inhibierendes Mittel, insbesondere als MMP-, Collagenase- und/oder Elastase-inhibierendes Mittel.
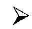 als Mittel mit einer Anti-Glykosylierungs-Aktivität, insbesondere gegen die Glykosylierung von kutanen Proteinen und bevorzugt gegen die Glykosylierung von Kollagen;
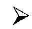 in Haarpflegemitteln, insbesondere zur Verbesserung der Kämmbarkeit.

### Pflegemittel:

Als Pflegemittel im Sinne der Erfindung sind Pflegemittel für Haut und Haar zu verstehen. Diese Pflegemittel schließen unter anderem reinigende und aufbauende Wirkung sowie UV-Lichtschutz Eigenschaften ein. Prinzipiell kann man die erfindungsgemäßen Extrakte in allen kosmetischen Produkten einsetzen. Beispiele für kosmetische Produkte sind in ihren Formulierungen in den Tabelle 10 bis Tabelle 13 beschrieben.

Die Haarpflege hat zum Ziel, den Naturzustand des frisch nachgewachsenen Haares möglichst lange zu erhalten bzw. bei Schädigung wiederherzustellen. Merkmale natürlichen gesunden Haares sind seidiger Glanz, geringe Porosität, spannkräftige und dabei weiche Fülle und angenehm glattes Gefühl (guter "Griff"). Die erfindungsgemäßen Pflegemittel wirken glättend auf das Haar, sie verbessern die Kämmbarkeit, vermindern die elektrostatatische Aufladung und verbessern Griff und Glanz.

Die erfindungsgemäßen Zubereitungen zeigen eine hervorragende hautpflegende Wirkung bei gleichzeitig hoher Hautverträglichkeit. Darüber hinaus zeigen sie eine gute Stabilität, insbesondere gegenüber oxidativer Zersetzung der Produkte.
Ein weiterer Gegenstand der Erfindung ist die Verwendung von Extrakten der Pflanze Cajanus cajan in Pflegemitteln mit lindernder, wohltuender und irritationshemmender Wirkung, insbesondere bei sensitiver und/oder geschädigter Haut und/oder Kopfhaut,

### Sonnenschutzmittel bzw. UV-Lichtschutzfaktoren

Ein weiterer Gegenstand der Erfindung ist die Verwendung der Extrakte der Pflanze Cajanus cajan in Sonnenschutzmittel.
Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) und UV-A (315-400 nm).

Die Pigmentierung normaler Haut unter dem Einfluss der Sonnenstrahlung, d. h. die Bildung von Melaninen, wird durch UV-B und UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnern. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u. gar Brandblasen führen kann.

Als UV-Absorber oder Lichtfilter, die also die UV-Strahlung in unschädliche Wärme umwandeln, werden Extrakte der Pflanze Cajanus cajan eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
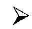 3-Benzylidencampher bzw. 3-Benzylidennorcampher und dessen Derivate, z.B. 3-(4-Methylbenzyliden)campher wie in der **EP 0693471 B1** beschrieben;
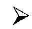 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
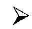 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
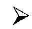 Ester der Salicylsäure, vorzugsweise Salicylsäure-2-ethylhexylester, Salicylsäure-4-isopropylbenzylester, Salicylsäurehomomenthylester;
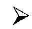 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
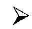 Ester der Benzalmalonsäure, vorzugsweise 4-Methoxybenzmalonsäuredi-2-ethylhexylester;
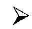 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
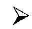 Propan-1,3-dione, wie z.B. 1-(4-tert.Butylphenyl)-3-(4'methoxyphenyl)propan-1,3-dion;
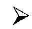 Ketotricyclo(5.2.1.0)decan-Derivate, wie in der **EP 0694521 B1** beschrieben.

Als wasserlösliche Substanzen kommen in Frage:
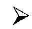 2-Phenylbenzimidazol-5-sulfonsäure und deren Alkali-, Erdalkali-, Ammonium-, Alkylammonium-, Alkanolammonium- und Glucammoniumsalze;
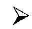 Sulfonsäurederivate von Benzophenonen, vorzugsweise 2-Hydroxy-4-methoxybenzophenon-5-sulfonsäure und ihre Salze;
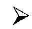 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze,

Als typische UVA-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parfümerie und Kosmetik 3 (1999), Seite 11ff** zu entnehmen.

In einer besonderen Ausführungsform der Erfindung werden die Extrakte der Pflanze Cajanus cajan in Mittel gegen die Schädigung menschlicher Hautzellen durch UV-Strahlung, insbesondere von Fibroblasten und/oder Keratinocyten durch UVA-Strahlung und/oder UVB-Strahlung und/oder als anti-inflammatorische Additive verwendet.

UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd (MDA) abgebaut, der viele biologische Moleküle wie Proteine und Nukleinbasen vernetzen wird (Enzymhemmung bzw. Mutagenese). Die erfindungsgemäßen Extrakte der Pflanze Cajanus cajan reduzieren signifikant den Grad an MDA in humanen Fibroblasten, welcher durch UVA-Strahlen induziert wird und zeigen damit eine hohe Kapazität schädliche Effekte eines oxidativen Stresses auf der Haut zu reduzieren.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasaenzyms LDH (Lactat Dehydrogenase) in humanen Keratinocyten dient als Marker für eine Zellschädigung.

Die erfindungsgemäßen Extrakte der Pflanze Cajanus cajan reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die Verwendung der erfindungsgemäßen Extrakte als anti-inflammatorische Additive ist prinzipiell für alle kosmetischen und/oder pharmazeutischen Zubereitungen möglich, die bei Entzündungen der Haut und damit in der Hautpflege eingesetzt werden. Die Entzündung der Haut kann dabei unterschiedlichste Ursachen haben.

### Antioxidantien bzw. Radikalfänger

Als Antioxidantien im Sinne der Erfindung sind Oxidationsinhibitoren zu verstehen, die sich aus der Pflanze Cajanus cajan isolieren lassen. Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Neben der Verwendung von Extrakten der Pflanze Cajanus cajan als Antioxidantien können auch weitere, bereits bekannte Antioxidantien eingesetzt werden. Eine mögliche Anwendung der Antioxidantien zum Beispiel in kosmetischen und/oder pharmazeutischen Zubereitungen, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt. Neben dem erfindungsgemäßen Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, Boldin, Boldo-Extrakt, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die weiteren UV-Lichtschutzfaktoren bzw. weiteren Antioxidantien können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Ein weiterer Gegenstand der Erfindung ist die Verwendung von Extrakten aus Cajanus cajan als Mittel gegen Rosacea. Bei Rosacea handelt es sich um chronisch bedingte Hautveränderungen deren Ursache bis heute nicht bekannt ist. Die Erscheinungsformen auf der Haut sind sehr unterschiedlich. In den meißten Fällen erscheint es als andauernde Rötung der Haut, v.a. der Gesichtshaut. Rosacea führt zu Hautunebenheiten und Hautausschlag.

Ein weiterer Gegenstand der Erfindung ist die Verwendung von Extrakten aus Cajanus cajan als Mittel gegen hormonell und/oder bakteriell bedingte Hautveränderungen, insbesondere gegen Akne.

Neben Rosacea gibt es noch viele weitere unerwünschte Hautveränderungen, die beispielsweise hormonell oder bakteriell bedingt sind. Zu diesen Hautveränderungen zählen im Sinne der Erfindung insbesondere alle Arten der Akne.

### Hautalterung

Die erfindugnsgemäßen Mittel und Extrakte aus *Cajanus cajan* wirken weiterhin gegen Hautalterung und können zur vorbeugenden oder heilenden Behandlung von Alterserscheinungen der Haut verwendet werden. Eine andere Bezeichnung für diese Art der Pflegemittel ist auch anti-ageing Mittel. Zu diesen Alterserscheinungen zählen beispielsweise jede Art der Fältchen- und Faltenbildung. Die Behandlungen schließen eine Verlangsamung von Altersprozessen der Haut mit ein. Die Alterserscheinungen können die unterschiedlichsten Ursachen aufweisen. Insbesondere sind diese Alterserscheinungen auf Grund einer durch UV-Strahlung induzierten Schädigung der Haut verursacht.

### Proteaseinhibierung

Die erfindungsgemäßen Extrakte aus Cajanus cajan wirken als Protease-inhibierendes Mittel, insbesondere als MMP- und/oder Collagenase- und/oder Elastase-inhibierendes Mittel. Unter MMP versteht man Matrix-Metallo-Proteasen. Zu den Matrix-Metallo-Proteasen zählen u.a. Collagenase, aber auch eine bestimmte Art der Elastasen. Die Aktivität der Enzyme ist abhängig von Metallionen - häufig handelt es sich um Zn²⁺-Ionen. Bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende Elastin abbauende Elastase ausgeschüttet. Des weiteren werden bei älteren Menschen oder nach UV-Strahlung durch die dermalen Fibroblasten interstitial Collagenasen - oder auch MMP-1 genannt-ausgeschüttet.

Diese Proteasen (Collagenase und die unterschiedlichen Elastasen) katalysieren die Fragmentierung und Zerstörung der dermalen Makromoleküle wie Proteoglycan, Collagen und Elastin. Sie verursachen damit eine Schwächung des Bindegewebes und führen dadurch zur Alterung der Haut und zu den Effekten der natürlichen Hautalterung nach UV-Strahlung.

Die hauptsächlich vorkommende Elastase zählt zur Gruppe der Serin-Proteasen. Ihre katalytische Reaktion beruht auf einen anderen Mechanismus. Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung

### Anti-Glykosylierung

Die erfindungsgemäßen Mittel und Extrakte aus *Cajanus cajan* besitzen eine Anti-Glykosylierungs-Aktivität, insbesondere wirken sie gegen die Glykosylierung von kutanen Proteinen und bevorzugt gegen die Glykosylierung von Kollagen. Im Jahre 1981 **(Science, 1981, 211, 491-493)** beschrieb A. Cerami die Glykosylierung von Proteinen oder nichtenzymatische Glykosylierung im Unterschied zur enzymatischen Glykosylierung durch Glukosyltransferase und erwähnte die mögliche Rolle dieser Glykosylierung bei der Alterung von Geweben, Der biochemische Mechanismus dieser Reaktion ist gut bekannt (**Borel J.P. et al., CR biologie prospective, 145-149,1993**) und läuft in zwei Phasen ab:

In einer frühen Phase reagieren zunächst reduzierende Zucker (Glucose, Fructose) mit den end- oder seitenständigen Aminofunktionen der im Gewebe enthaltenen Proteine zu sogenannten Schiffschen Basen. Diese Verbindungen werden dann durch Amadori-Umlagerung zum Ketoamin stabilisiert.

Die Ketoaminfunktionen werden dann in einer späten Phase in Gegenwart von Sauerstoff zur Desoxyonose oxidiert und reagieren mit zu anderen Proteinen (Albumin, Lipoproteinen, Immunoglobulin) gehörigen anderen basischen Aminosäuren wie Arginin oder Lysin. Dabei bilden sich Komplexe, die letztendlich über Pentosidin- oder 2-Furoyl-1,4-imidazol-Cyclen verbrückt sind. Durch diese Verbrückungen ergeben sich als komplexe und sehr stabile Endprodukte die sogenannten AGE ("Advanced Glycosylation Endproducts") oder fortgeschrittene Produkte der Glykosylierung. Diese späte Phase ist sehr langsam und irreversibel.

Die Glycosylierung der Proteine führt zur Bildung von inter- und intramolekularen Brücken bei langsam erneuerten Proteinen und schließlich zur Braunfärbung und Unlöslichkeit dieser Proteine. Die Glykosylierung betrifft insbesondere die Proteine in den extrazellulären Matrizes, deren Erneuerung langsam ist.

Im Bereich der Haut handelt es sich bei den durch die Glycosylierung geschädigten Proteinen insbesondere um Fibronektin, Laminin, Elastin und die verschiedenen Collagentypen.

Die AGE führen zu verschiedenen Beschwerden:
- da sie voluminös sind, bleiben die Moleküle, die sie tragen, nur schwerlich an ihrem normalen Platz,
- die glycosylierten Moleküle büßen ihre Flexibilität ein (Gewebeversteifung),
- die glycosylierten Moleküle können gegenüber Enzymen, die deren Erneuerung gewährleisten, widerstandsfähiger werden und so Flächen aus amorphen Substanzen bilden.

In normaler Haut werden die glykosylierten Proteine auf metabolischem und zellulärem Wege eliminiert, insbesondere durch Abbau durch Makrophagen, was eine Neugestaltung der Dermis induziert.

Diese Eliminierung läßt jedoch mit zunehmendem Alter nach, was eine Anreicherung dieser glykosylierten Proteine und eine beschleunigte und verstärkte Alterung der Dermis zur Folge hat, wofür die Summe mehrerer Phänomene verantwortlich ist:
- Beständigkeit gegenüber Erneuerungsproteasen und Abnahme der Fibrillogenese und damit der Erneuerung von Kollagen, Verminderung seiner Filterwirkung in der extrazellulären Matrix und insbesondere am Dermis-Epidermis-Übergang nach Fixierung von Fremdproteinen (LDL, Cholesterin, Albumin), was zu Verdickung führt,
- die glykosylierten Proteine stellen eine potentielle Quelle freier Sauerstoffradikale dar. Dieses durch UV-A verstärkte Phänomen führt zum Collagenabbau,
- Aktivierung von unspezifischen, schädlichen Proteasen,
- Aktivierung von Makrophagen und Ausschüttung von Cytokinen (TNF-alpha),
- schließlich Entzündung mit anschließender Fibrose und Lipofuszin-Ablagerung.

Die obigen Ausführungen sind für die Verwendung von Substanzen mit Anti-Glykosylierungs-Aktivität, insbesondere bei der Bekämpfung und Prävention der Haut- und insbesondere der Haaralterung in der Kosmetik, von Interesse. Die Unterdrückung der nichtenzymatischen Glykosylierung stellt unter anderem ein wichtiges Ziel zur Verhinderung von Haarausfall dar.

Studien haben gezeigt, dass eine erhöhte Exposition der Haut mit UV-Strahlung zu einer Erhöhung der Glykosylierung führt. Da die Kopfhaut einer extrem hohen Belastung mit UV-Strahlung ausgesetzt ist und die Glykosylierung direkt mit dem Fortschreiten des Haarausfalls korreliert, können Mittel, die einer Glykosylierung entgegenwirken direkt gegen Haarausfall eingesetzt werden. Zeigen diese Mittel wie es für die erfindungsgemäßen Mittel nachgewiesen wurde zusätzlich noch UV/IR-Lichtschutzwirkung, so erfüllen sie hier den gewünschten vielfältigen Effekt.

Die erfindungsgemäßen Mittel enthaltend Extrakte aus Cajanus cajan verbessern die Kämmbarkeit des Haares und insbesondere die Kämmbarkeit bei trockenem Haar. Diese Aktivität erlaubt die Verwendung in jeglichen Haarpflegeprodukten wie beispielsweise Haarshampoo, Haarlotion, Haarspülung, Haarkur, Haarfestiger und insbesondere den Einsatz in kombinierten Präparaten die eine tägliche Anwendung am nassen oder am trockenen Haar sowie an gebleichtem oder dauergewelltem, geschädigtem Haar erlaubt.

Ein weiterer Gegenstand der Erfindung ist ein Verfahren zur Herstellung eines Extraktes der Pflanze Cajanus cajan, bei dem zur Extraktion Lösungsmittel oder Mischungen dieser Lösungsmittel verwendet werden, welche ausgewählt sind aus der Gruppe, die gebildet wird von destilliertem oder nicht destilliertem Wasser, niedermolekularen Alkoholen, Estern Kohlenwasserstoffe, Ketone oder halogenhaltige Kohlenwasserstoffe und der so erhaltene Extrakt gegebenenfalls getrocknet wird. Bevoruzgt ist ein Verfahren bei dem zur Extraktion destilliertes Wasser, Methanol, Ethanol oder Gemische aus Wasser und Methanol oder Wasser und Ethanol eingesetzt werden.

Die erfindungsgemäßen Extrakte können in kosmetischen und/oder pharmazeutischen Zubereitungen, wie beispielsweise Haarshampoos, Haarlotionen, Schaum-bäder, Duschbäder, Cremes, Gele, Lotionen, Sonnenschutzmittel, alkoholische und wäßrig/alkoholische Lösungen, Emulsionen, Wachs/Fett-Massen, Stiftpräparaten, Pudern oder Salben eingesetzt werden. Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe und dergleichen enthalten.

Als Hilfs- und Zusatzstoffe mit Oberflächenaktivität können anionische, nichtionische, kationische und/oder amphotere bzw. amphotere **Tenside** enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisothionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat in Betracht. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1),** insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane.

Als **Emulgatoren** kommen beispielsweise nichtionogene Tenside aus mindestens einer der folgenden Gruppen in Frage:
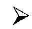 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
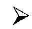 Alkyl- und/oder Alkenyloligoglykoside mit 8 bis 22 Kohlenstoffatomen im Alk(en)ylrest und deren ethoxylierte Analoga;
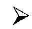 Anlagerungsprodukte von 1 bis 15 Mol Ethylenoxid an Ricinusöl und/oder gehärtetes Ricinusöl;
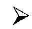 Anlagerungsprodukte von 15 bis 60 Mol Ethylenoxid an Ricinusöl und/oder gehärtetes Ricinusöl;
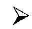 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
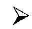 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
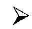 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
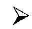 Mono-, Di- und Trialkylphosphate sowie Mono-, Di- und/oder Tri-PEG-alkylphosphate und deren Salze;
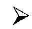 Wollwachsalkohole;
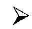 Polysiloxan-Polyalkyl-Polyether-Copolymere bzw. entsprechende Derivate;
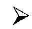 Block-Copolymere z.B. Polyethylenglycol-30 Dipolyhydroxystearate;
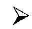 Polymeremulgatoren, z.B. Pemulen-Typen (TR-1 ,TR-2) von Goodrich;
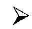 Polyalkylenglycole sowie
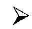 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Typische Beispiele für **Fette** sind Glyceride, d.h. feste oder flüssige pflanzliche oder tierische Produkte, die im wesentlichen aus gemischten Glycerinestern höherer Fettsäuren bestehen, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z.B. Magnesium-, Aluminium- und/oder Zinkstearat bzw. -ricinoleat eingesetzt werden.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840** A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosmetics & Toiletries Vol. 108, Mai 1993, Seite 95ff** aufgeführt.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt, Eine detaillierte Übersicht über geeignete flüchtige Silicone findet sich zudem von Todd et al. in **Cosm.Toil. 91, 27 (1976).**

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, weitere Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Tridosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren sind** beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat,

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame, Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
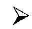 adstringierende Wirkstoffe,
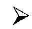 Ölkomponenten,
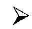 nichtionische Emulgatoren,
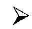 Coemulgatoren,
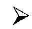 Konsistenzgeber,
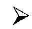 Hilfsstoffe wie z. B. Verdicker oder Komplexierungsmittel und/oder
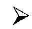 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
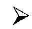 entzündungshemmende, hautschützende oder wohlriechende ätherische Öle,
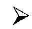 synthetische hautschützende Wirkstoffe und/oder
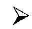 öllösliche Parfümöle

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Antischuppenwirkstoffe** kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Als **Insekten-Repellentien** kommen N,N-Diethyl-m-toluamid, 1,2-Pentandiol oder Ethyl Butylacetylaminopropionate in Frage

Als **Selbstbräuner** eignet sich Dihydroxyaceton. Als Tyrosinaseinhibitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
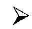 Glycerin;
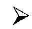 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
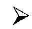 technische Oligoglyceringemische mit einem Eigenkondensationsgrad von 1,5 bis 10 wie etwa technische Diglyceringemische mit einem Diglyceringehalt von 40 bis 50 Gew.-%;
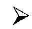 Methyolverbindungen, wie insbesondere Trimethylolethan, Trimethylolpropan, Trimethylolbutan, Pentaerythrit und Dipentaerythrit;
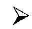 Niedrigalkylglucoside, insbesondere solche mit 1 bis 8 Kohlenstoffen im Alkylrest, wie beispielsweise Methyl- und Butylglucosid;
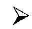 Zuckeralkohole mit 5 bis 12 Kohlenstoffatomen, wie beispielsweise Sorbit oder Mannit,
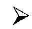 Zucker mit 5 bis 12 Kohlenstoffatomen, wie beispielsweise Glucose oder Saccharose;
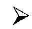 Aminozucker, wie beispielsweise Glucamin;
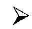 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax), Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Zubereitung, eingesetzt.

### Beispiele

### Beispiel 1:

100 g zerkleinerte Blätter von Cajanus cajan Pflanzen aus Indien wurden in einen Glasreaktor überführt und mit 2 l destilliertem Wasser aufgegossen. Der Aufguß wurde zwischen 80 und 90 °C erhitzt und unter Rühren über einen Zeitraum von 1 h bei dieser Temperatur extrahiert. Anschließend wurde die Mischung auf 20 °C abgekühlt und 15 min bei einer Geschwindigkeit von 5000 g zentrifugiert. Die überstehende kolloide Flüssigkeit wurde durch Filtration an Tiefenfilter mit einer mittleren Porosität von 0,450 µm (von der Firma Seitz, Bordeaux Frankreich) vom Rückstand getrennt. Der Extrakt wurde bei einer Anfangstemperatur von 185°C und einer Endtemperatur von 80°C sprühgetrocknet. Die Ausbeute an Trockenprodukt betrug 17 Gew.-% bezogen auf das Trockengewicht an eingesetzten Pflanzen.

### Beispiel 2:

Beispiel 1 wurde wiederholt, die Extraktion jedoch mit 300 g zerkleinerte Blätter der Pflanze Cajanus cajan aus Indien in 3 | 80 Gew.-%-igem wässrigen Methanol durchgeführt. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt. Anschließend wurde zunächst der Alkohol bei 30 °C unter vermindertem Druck entfernt und dann der wäßrige Rückstand wie beschrieben sprühgetrocknet. Die Extraktion wurde an zwei Proben oder Batches (Batch A und Batch B) von Ausgangsmaterial durchgeführt. Die Ausbeute an Trockenprodukt betrug 12,4 Gew.-% für Batch A und 10,9 Gew.-% für Batch B bezogen auf das Trockengewicht an eingesetzten Pflanzen.

### Beispiel 3:

Beispiel 1 wurde wiederholt, die Extraktion jedoch mit 1,9 60 Gew.-%-igem wässrigen Ethanol durchgeführt. Die Extraktion wurde unter Rühren 1 h bei einer Temperatur zwischen 50 und 65 °C durchgeführt und der Extrakt wie beschrieben weiter verarbeitet. Die Filtration wurde wie im Beispiel 1 beschrieben durchgeführt. Anschließend wurde zunächst der Alkohol bei 45 °C unter vermindertem Druck entfernt und dann der Rückstand wie in Beispiel 1 beschrieben getrocknet. Die Ausbeute an Trockenprodukt betrug 12,5 Gew.-% bezogen auf das Trockengewicht an eingesetzten Pflanzen.

### Beispiel 4: Aktivität gegenüber freien Radikalen.

In einer ersten Testreihe wurde die Eignung der Extrakte gegen oxidativen Stress durch chemische Methoden untersucht. Eingesetzt wurden die Extrakte gemäß Beispiel 2. Als erstes Testsubstrat wurde Diphenylpicrylhydrazyl (DPPH) gewählt, ein purpurrot gefärbtes stabiles Radikal, welches durch Inkontaktbringen mit Radikalfängern in sein ungefärbtes Leucoderivat übergeht. Der Farbwechsel wurde photometrisch bei 513 nm verfolgt. Die Meßergebnisse sind in Tabelle 1 zusammengefaßt ("DPPH-Test"), angegeben ist die Inhibierung von DPPH in %-absolut. In einem weiteren Test wurde als Referenzsystem die Hydroxylierung von Salicylsäure durch Hydroxylradikale (aus der Reaktion von Wasserstoffperoxid mit Eisen(III)ionen und EDTA) untersucht. Auch diese Reaktion kann photometrisch untersucht werden, da das Hydroxylierungsprodukt rötlich gefärbt ist. Gemessen wurde der Einfluß der Extrakte auf die Bildung der Hydroxysalicylsäure bei einer optischen Dichte von 490 nm. Die Meßergebnisse sind ebenfalls in Tabelle 1 zusammengefaßt, angegeben ist wieder die Inhibierung in %-absolut ("Salicylsäure-Test"). In einem dritten und letzten Test wurde Desoxyribose als Testsubstanz eingesetzt. In Gegenwart von Hydroxylradikalen, welche aus H₂O₂ in Gegenwart von Fe²⁺ und EDTA gebildet werden wird Desoxyribose oxidiert. Durch Kondensation mit Thiobarbitursäure entsteht aus der oxidierten Form eine pinkfarbige Substanz. Die optische Dichte wurde bei 532 nm bestimmt und ist abhängig vom Gehalt an oxidierter Desoxyribose. Eine radikalfangende Substanz reagiert mit den entstehenden Hydoxylradikalen und reduziert die Bildung an pinkfarbigen Substanzen bei dieser Reaktionsmischung. Die gleiche Reaktion wurde außerdem noch ohne EDTA durchgeführt um komplexbildende Eigenschaften des Extraktes mit Fe²⁺ Ionen zu untersuchen. Die Ergebnisse der letzten beiden Untersuchungen sind in Tabelle 2 zusammengefaßt.

**Tabelle 1:**

| **Radikalfangende Eigenschaften in % absolut (Ergebnisse sind Mittelwerte aus zwei Messungen)** | | | |
|---|---|---|---|
| | **DPPH-Test** | **Salicylsäure-Test** | |
| | Extrakt nach Beispiel 2 | Extrakt nach Beispiel 2 | |
| **Konzentration [Gew.-%]** | Batch A | Batch A | Batch B |
| Kontrolle | 0 | 0 | 0 |
| 0,001 | 30 | - | - |
| 0,01 | 68 | - | - |
| 0,03 | 90 | 33 | 11 |
| 0,1 | 92 | 61 | 43 |
| IC50 in % [Gew.-%] | 0,0068 | 0,0725 | 0,115313 |

**Tabelle 2:**

| **Radikalfangende Eigenschaften mit Desoxyribose % absolut (Ergebnisse sind Mittelwerte aus zwei** Messungen) | | | | |
|---|---|---|---|---|
| | **Reaktion mit EDTA** | | **Reaktion ohne EDTA** | |
| | Extrakt nach Beispiel 2 | | Extrakt nach Beispiel 2 | |
| **Konzentration [Gew.-%]** | Batch A | Batcn B | Batch A | Batch B |
| Kontrolle | 0 | 0 | 0 | 0 |
| 0,03 | 16 | 10 | 19 | 8 |
| 0,1 | 38 | 31 | 39 | 43 |
| IC50 in % [Gew.-%] | 0,138182 | 0,163333 | 0,137 | 0,114 |

Aus den Werten der Tabelle 1 und Tabelle 2 lässt sich erkennen, dass die verwendeten Extrakte der Pflanze Cajanus cajan eine Wirkung gegen Radikale zeigen.

In einem weiteren Test wurden die radikalfangenden Eigenschaften von Extrakten aus Cajanus cajan in einem Test mit Xanthin Oxidase als Testsystem untersucht. Das Enzym bewirkt bei oxidativem Stress die Umwandlung von Purinbasen, wie z.B. Adenin oder Guanin in Uronsäure, wobei die intermediär gebildeten superoxid Sauerstoffradikale gebildet werden, welche durch die Superoxid-Dismutase (SOD) spontan in H₂O₂ und Sauerstoff zerfallen. Diese Superoxid-Radikale können durch Reaktion mit Luminol, oder Luminol und Microperoxidase und durch NBT über die Lumineszenz und die optische Dichte bei 490 nm nachgewiesen und quantitativ bestimmt werden. In Gegenwart von Substanzen mit radikalfangenden Eigenschaften vermindert sich die Lumineszenzausbeute. Diese Ergebnisse sind in Tabelle 3 zusammengefasst; wieder ist die Inhibierung in %-absolut angegeben ("Luminol-Test").

**Tabelle 3:**

| **Test mit Xanthin-Oxidase in % absolut** | | | | | | |
|---|---|---|---|---|---|---|
| | **Luminol** | | **Luminol + Microperoxidase** | | **NBT** | |
| | Extrakt nach Beispiel 2 | | Extrakt nach Beispiel 2 | | Extrakt nach Beispiel 2 | |
| **Konzentration [Gew.-%]** | Batch A | Batch B | Batch A | Batch B | Batch A | Batch B |
| Kontrolle | 0 | 0 | 0 | 0 | 0 | 0 |
| 0,001 | 45 | 16 | | | | |
| 0,01 | 92 | 71 | 0 | 8 | | |
| 0,03 | | | 57 | 51 | 21 | 22 |
| 0,1 | | | 94 | 98 | 46 | 53 |
| IC50 in % | 0,0020 | 0,0020 | 0,0275 | 0,029535 | 0,1112 | 0,0932 |

Durch die Ergebnisse die in den Tabellen 1 bis 3 zusammengefasst wurden, lässt sich zeigen, dass Extrakte aus den Blättern von Cajanus cajan sehr gute radikalfangende Eigenschaften besitzen. Bereits bei einer Konzentration von 0,001 Gew.-% zeigten sich radikalfangende Eigenschaften, beim Test mit DPPH und führten zu einer 30 %igen Entfärbung der Lösung im Vergleich zur Kontrolle. Bei einer Konzentration von 0,1 Gew.-% führte dieser Test bereits zu einer 92 %igen Entfärbung. Es wurden also 92% der vorhandenen DPPH-Radikale "eingefangen". Im Test mit Desoxyribose zeigte sich nicht nur radikalfangende Eigenschaften, sondern auch die Fähigkeit mit Fe²⁺-lonen Komplexe zu bilden. Im Test mit Luminol zeigte sich erneut eine sehr gute radikalfangende Eigenschaft.

### Beispiel 5: Toxizität

Das Ziel dieser Tests ist die Bestimmung der toxischen Konzentration des zu untersuchenden Mittels für Fibroblasten zur besseren Bestimmung der am besten wirksamen Konzentration. Der noch wirksame Konzentrationsbereich kann damit eingegrenzt werden.

Methode: Effekte am Zellwachstum Humane Fibroblasten wurden in einem definiertem Nährmedium (DMEM = Dulbecco Minimum Essential Medium, Firma Life Technologie Sarl) mit 10 Gew.-% fötalem Kälberserum angeimpft und für 24h bei 37°C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche Konzentrationen an Pflanzenextrakt gegeben. Nach einer drei tägigen Inkubation der Fibroblasten im Nährmedium wurde das Wachstum und die Stoffwechselaktivität beurteilt, indem der intrazelluläre Anteil an ATP nach der enzymatischen Lumineszenz-Methode von Vasseur (*Journal francais Hydrologie,* **1981,** 9, 149-156.) und der Anteil an Zellproteinen nach der Methode von Bradford *(Anal. Biochem.,* **1976,** 72, 248-254) bestimmt wurde.

Die Bestimmung des Anteils an zellulären Proteinen erlaubt einen Rückschluß auf den Anteil an Makromolekülen wie Enzymen, Collagen, Elastin oder anderen dermalen Makromolekülen, die zur Bildung von Bindegewebe notwendig sind. Der ATP-Anteil einer Zelle ist wichtig für viele Enzyme, deren Aktivität von diesem Energieüberträger abhängig ist,

Bestimmt wurde die letale Dosis des Extraktes aus Cajanus cajan bei der 50 % der untersuchten Fibroblasten nicht mehr lebensfähig waren (LD 50). Bis zu einer Konzentration von **0,005** Gew.-% zeigt der Extrakt aus Cajanus cajan keine toxischen Effekte auf die humanen Fibroblasten.

### Beispiel 6: Zellschutzwirkung gegen UVA an in vitro gezüchteten menschlichen Fibroblasten

Hintergrund: UVA-Strahlen dringen bis in die Dermis ein, wo sie zu Oxidationsstreß führen, was durch eine Lipoperoxidation der Zytoplasmamembranen nachgewiesen wird.

Die Lipoperoxide werden zu Malonaldialdehyd abgebaut, der viele biologische Moleküle wie Proteine und Nukleinbasen vernetzen wird (Enzymhemmung bzw. Mutagenese).

Methode: Zur Durchführung dieser Tests wurde ein definiertes Kulturmedium (DMEM) mit den Fibroblasten mit fötalem Kälberserum beimpft und der Pflanzenextrakt (in dem definierten Medium mit 10 % Fötalem Kälberserum) 72 Stunden nach dem Beimpfen zugegeben.

Nach 48 stündiger Inkubation bei 37 °C und einem CO₂-Gehalt von 5 % wurde das Kulturmedium durch Saline-Lösung (physiologische NaCI-Lösung) ersetzt und die Fibroblasten wurden mit einer UVA-Dosis bestrahlt (365 nm, 3-15 J/cm²; Röhren: MAZDA FLUOR TFWN40).

Nach der Beendigung der Bestrahlung wurde der MDA-Spiegel (Malonaldialdehyd-Spiegel) in der überstehenden Natriumchlorid-Lösung quantitativ durch Reaktion mit Thiobarbitursäure bestimmt. Der Gehalt an Proteinen wurde nach der Methode von Bradford bestimmt.

| **Konzentration [Gew.-%]** | **Gehalt an MDA [% versus Kontrolle]** | **Gehalt an Proteinen [% versus Kontrolle]** |
|---|---|---|
| | Extrakt nach Beispiel 1 | Extrakt nach Beispiel 1 |
| Kontrolle ohne UV | 0 | 100 |
| UVA (365 nm) | 100 | 102 |
| UVA + Extrakt 0,1 % | 76 | 138 |
| UVA + Extrakt 0,3 % | 46 | - |

| **Konzentration [Gew.-%]** | **Gehalt an MDA [% versus Kontrolle]** | | **Gehalt an Proteinen [% versus Kontrolle]** | |
|---|---|---|---|---|
| | Extrakt nach Beispiel 2 | | Extrakt nach Beispiel 2 | |
| | Batch A | Batch B | Batch A | Batch B |
| Kontrolle ohne UVA | 0 | 0 | 100 | 100 |
| Kontrolle mit UVA | 100 | 100 | 100 | 97 |
| UVA + Extrakt 0,002 % | 66 | 74 | 101 | 103 |
| UVA + Extrakt 0,005 % | 60 | 50 | 93 | 110 |

**Tabelle 4:**

| **Quantifizierung von Malonaldialdehyd in Fibroblasten (Ergebnisse in % bezogen auf die Kontrolle,** Mittelwert aus 2 Versuchen, jeder mit drei Wiederholungen | | |
|---|---|---|
| **Konzentration [Gew.-%]** | **Gehalt an MDA [% versus Kontrolle]** | **Gehalt an Proteinen [% versus Kontrolle]** |
| | **Extrakt nach Beispiel 1** | **Extrakt nach Beispiel 1** |
| Kontrolle ohne UV | 0 | 100 |
| UVA (365 nm) | 100 | 102 |
| UVA + Extrakt 0,01 % | 51 | 120 |
| UVA + Extrakt 0,03 % | 43 | 123 |

Die Ergebnisse aus der Tabelle 4 zeigen, dass die Extrakte der Pflanze Cajanus cajan signifikant den Grad an MDA in humanen Fibroblasten, welcher durch UVA-Strahlen induziert wird, reduzieren. Diese Ergebnisse zeigen eine hohe Kapazität von Cajanus cajan Extrakten schädliche Effekte eines oxidativen Stresses auf der Haut zu reduzieren. Der Gehalt an Proteinen verdeutlicht nochmals den nichttoxischen Effekt des Extraktes.

### Beispiel 7: Zellschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, welche Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium (DMEM), das fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm² - Röhren: DUKE FL40E).

Nach weiterer 1 tägiger Inkubation bei 37 °C und bei 5 % CO₂ wurde der LDH-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche). Der Gehalt an PGE2 wurde mit einem ELISA-Test (ELISA Kit der Firma Roche) bestimmt. Die Anzahl adhärenter Keratinozyten wird (nach Trypsinbehandlung) mit einem Partikelzählgerät bestimmt.

| Extrakt nach Beispiel 1 [Gew.-%] | Anzahl Keratinocyten | Gehalt an freigesetzte LDH | Gehalt an PGE2 |
|---|---|---|---|
| Kontrolle ohne UV | 100 | 0 | 0 |
| UVB (315 nm) | 38 | 100 | 100 |
| UVB + Extrakt 0,03 % | 45 | 77 | - |
| UVB + Extrakt 0,1 % | 89 | 27 | 7 |
| UVB + Extrakt 0,3 % | 134 | 4 | 2 |

| Extrakt nach Beispiel 2 [Gew.-%] | Anzahl Keratinocyten | | Gehalt an freigesetzte LDH | | Gehalt an PGE2 | |
|---|---|---|---|---|---|---|
| | Batch A | Batch B | Batch A | Batch B | Batch A | Batch B |
| Kontrolle ohne UV | 100 | 100 | 0 | 0 | 0 | 0 |
| UVB (315 nm) | 54 | 47 | 100 | 100 | 100 | 100 |
| UVB + Extrakt 0,002 % | 72 | 76 | 66 | 22 | 61 | 50 |
| UVB + Extrakt 0,005 % | 91 | 147 | 38 | 0 | 36 | 7 |

**Tabelle 5 :**

| **Zellschutzwirkung der Extrakte von Cajanus cajan gegen UVB-Strahlen; Ergebnisse in % bezogen auf die Kontrolle, Mittelwert aus 2 Versuchen, jeder mit zwei Wiederholungen** | | | |
|---|---|---|---|
| Extrakt nach Beispiel 3 [Gew.-%] | Anzahl Keratinocyten | Gehalt an freigesetzte LDH | Gehalt an PGE2 |
| Kontrolle ohne UV | 100 | 0 | 0 |
| UVB (315 nm) | 38 | 100 | 100 |
| UVB + Extrakt 0,003 % | 45 | 70 | - |
| UVB + Extrakt 0,01 % | 86 | 29 | 21 |
| UVB + Extrakt 0,03 % | 148 | 0 | 10 |

Die Ergebnisse dieser Tests belegen, dass die Extrakte der Pflanze Cajanus cajan den Effekt von UVB-Strahlung auf die Anzahl an Keratinocyten, auf den Gehalt an freigesetzte LDH und auf den Gehalt an PGE2 positiv beeinflussen. Die beschriebenen Extrakte zeigen demnach die Fähigkeit, die durch UVB-Strahlung hervorgerufene Schädigung an Zellmembranen zu reduzieren.

### Beipsiel 8: Anti-Protease -

Bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende Elastin abbauende Elastase ausgeschüttet. Des weiteren werden bei älteren Menschen oder nach UV-Strahlung durch die dermalen Fibroblasten interstitial Collagenasen - oder auch MMP-1 genannt - ausgeschüttet.

Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung.

### 8a. Inhibierung der Elastase-Aktivität

Serin-Proteasen, wie z.B. eine Art der Elastase, bewirken den Abbau von Elastin, Proteoglycanen und Kollagen und verursachen damit eine Schwächung des Bindegewebes. Im folgenden Test wurden die inhibierenden Eigenschaften des Extraktes nach Beispiel 2 an einem chromogenen synthetischen Substrat (bezogen von SIGMA) untersucht. Die Inkubationszeit betrug 30 min bei Raumtemperatur. Die Inhibierung wurde photometrisch bei 410 nm verfolgt, als Positiv-Standard diente α1-Antitrypsin. Die Ergebnisse sind in Tabelle 6 zusammengefasst.

**Tabelle 6:**

| **Inhibierung der Elastase (Ergebnisse in % Inhibierung)** | | |
|---|---|---|
| **Konzentration [Gew.-%]** | **Extrakt nach Beispiel 2** | |
| | Batch A | Batch B |
| 0,15 | 50 | - |
| 0,3 | 92 | 35 |

Der Extrakt zeigt damit eine Aktivität gegen die Serin-Protease Elastase. Diese Aktivität führt zum möglichen Einsatz gegen Effekte der Hautalterung, die in einer erhöhten Aktivität der Serin-Protease Elastase begründet liegen.

### 8b. Inhibierung der Collagenase

Dermale Fibroblasten älterer Menschen schütten nach Sonnenexposition Collagenasen - auch Matrix Metallo-Protease-1 (MMP-1) - aus. In einem ersten Test wurde Collagenase aus *Clostridium histolyticum* untersucht, welche mit chromogenen synthetischen Substrat FALGPA markiert worden war. Die Inkubationszeit betrug 30 min bei Raumtemperatur. Die Hydrolyse von Collagen wurde photometrisch bei 324 nm bestimmt. Als Vergleichssubstanz diente Cystein oder EDTA.

**Tabelle 7 :**

| **Inhibierung der Collagenase (Ergebnisse in % Inhibierung)** | | |
|---|---|---|
| **Konzentration [Gew.-%]** | **Extrakt nach Beispiel 2** | |
| | Batch A | Batch B |
| 0,23 | - | 50 |
| 0,3 | 27 | 78 |

Die Ergebnisse belegen den inhibierenden Effekt der Extrakte aus Cajanuns cajan gegen die MMP-Collagenase. Diese Aktivität führt zu dem Einsatz gegen Effekte der Hautalterung, die aufgrund eines Abbaus der dermalen Makromoleküle Collagen auftreten.

### Beipsiel 9: Inhibierung der Glykosylierung von Collagen

Zum Nachweis, dass die Extrakte von Cajanus cajan die nicht-enzymatische Glykosylierung von Makromolekülen inhibieren, wurde Collagen des Typs 1 mit Glucose und den Extrakten über einen Zeitraum von 21 Tagen bei 45 °C behandelt. Anschließend wurden die Suspensionen zentrifugiert und der Gehalt an Schiff'schen Basen in der überstehenden Flüssigkeit durch Fluoreszenzmessung bei 430 nm bestimmt. Die Ergebnisse sind in Tabelle 7 zusammengefasst. Die Angaben beziehen sich auf die Kontrolle als Standards (ohne Extrakt und ohne Glucose).

**Tabelle 8:**

| **Ausbeute an Schiff'schen Basen** | | | | |
|---|---|---|---|---|
| **Konzentration [Gew.-%]** | **Extrakt nach Beispiel 1** | **Extrakt nach Beispiel 2** | | **Extrakt nach Beispiel 3** |
| | | **Batch A** | **Batch B** | |
| Kontrolle ohne Glucose | 22,9 | 3,6 | 38,4 | 22,9 |
| Kontrolle mit Glucose | 89,5 | 100 | 101,8 | 89,5 |
| Glucose und Extrakt 0,01 Gew.-% | - | 91,3 | 89,7 | - |
| Glucose und Extrakt 0,1 Gew.-% | - | 25,8 | 52,1 | - |
| Glucose und Extrakt 0,3 Gew.-% | 62,6 | | | 77,6 |
| Glucose und Extrakt 1 Gew.-% | 55 | | | 41,3 |
| Glucose und Extrakt 3 Gew.-% | 15,6 | | | 4,7 |

Die Ergebnisse belegen eine reduzierende Aktivität auf die nicht-enzymatische Glykosylierung von Collagen und damit einen reduzierenden Effekt auf die Alterung der Dermis.

### Beispiel 10: Sensorische Aktivität auf menschlichen Haaren

Die Bewertung der Modifikation sensorischer Eigenschaften an menschlichen Haaren nach der Behandlung mit Extrakten der Pflanze Cajanus cajan wurde an standardisierten Haarsträhnen (20 cm Länge und 5 g Gewicht) durchgeführt. Die Proben der Pflanzenextrakte wurden in einer Konzentration von 1,5 Gew.-% in ein Shampoo eingearbeitet und getestet. Die Kämmbarkeit wurde vor und nach der Behandlung mit dem Shampoo an dem getrocknetem Haar getestet. Vor Bestimmung der Kämmbarkeit und vor Behandlung mit dem Shampoo wurden die Haarstähnen mit wässrige Natrium-Lauryl-Sulfat Lösung (15 Gew.-%) gewaschen.

Zur Bestimmung der Kämmbarkeit wurden die Haasträhnen am oberen Ende fixiert und die Kraft die zum Kämmen aufgewendet werden muss in einem standardiesertem Gerät, unter klimatisierten Bedingungen gemessen, indem der Kamm mit gleichbleibender Geschwindigkeit durch die Strähne geführt wurde.

Als Standard wurde die Kämmbarkeit an trockenen Haarsträhnen bestimmt, welche über Nacht in einem klimatisierten Gehäuse bei 22 °C und einer relativen Luftfeuchtigkeit von 40 % gelagert worden waren. Drei Kämmbarkeitsversuche wurden an der selben Haarsträhne durchgeführt.

Die Behandlung der Haarsträhnen wurde mit 3 ml Shampoo, enthaltend den Extrakt aus Cajanus cajan über 3 min durchgeführt. Anschliessend wurde die Haarsträhne 1 min. mit Wasser ausgespühlt. Die so behandelte Haarsträhne wurde ebenfalls über Nacht bei 22 °C und 40 % Luftfeuchtigkeit in dem klimatisierten Gehäuse gelagert und dreimal mit den gleichen Kämmbarkeitsversuchen getestet.

Bestimmt wurde zum einen die höchste Kraft, die während des Kämmvorganges aufgewendet werden musste und zum anderen die gesamt Kämmarbeit, welche ausgedrückt wird durch den Mittelwert der gemessenen Kraft bei den jeweiligen Kämmbarkeitsversuchen.

**Tabelle 9:**

| **Bestimmung der Kraftaufwendung bei der Kämmbarkeit** | | | | |
|---|---|---|---|---|
| | Test 1 | | Test 2 | |
| | Placebo | Extrakt nach Beispiel 2 Batch A | Placebo | Extrakt nach Beispiel 2 Batch B |
| höchste Kraft | +8 | -28 | +11 | -34 |
| Kämmarbeit | +18 | -35 | 0 | -37 |

Die Extrakte von Cajanus cajan eignet sich zur Pflege von Haaren, da sie die Kämmbarkeit von trockenen Haaren verbessern.

Als praktische Ausführungsbeispiele der Erfindung, sollen im folgenden unterschiedliche Kosmetikprodukte oder Kosmetikpräparate beschrieben werden, die einen Extrakt der Pflanze Cajanus cajan enthalten.

In den nachfolgenden Tabellen sind eine Reihe von Formulierungsbeispielen angegeben.

### Beispiel 10: Beispielrezepturen kosmetischer Mittel mit Extrakten der Pflanzen Cajanus cajan

Die gemäß Beispiel 1 bis 3 gewonnenen Extrakte der Pflanze Cajanus cajan wurden in den folgenden erfindungsgemäßen Rezepturen K1 bis K21 sowie 1 bis 40 eingesetzt. Sofern nicht explizit anders angegeben ist, kann jeder Extrakt gemäß der Beispiele 1 bis 3 eingesetzt werden. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus erweisen sich die erfindungsgemäßen Mittel als stabil gegen oxidative Zersetzung.

**Tabelle 10:**

| **Softcreme Rezepturen K1 bis K7** | | | | | | | | |
|---|---|---|---|---|---|---|---|---|
| (Alle Angaben in Gew.-% bez. auf das kosmetische Mitteln) | | | | | | | | |

| INCI Bezeichnung | K1 | K2 | K3 | K4 | K5 | K6 | K7 | V1 |
|---|---|---|---|---|---|---|---|---|
| Glyceryl Stearate (and) Ceteareth-12/20 (and) Cetearyl Alcohol (and) Cetyl Palmitate | 8,0 | 8,0 | 8,0 | 8,0 | 8,0 | 8,0 | 8,0 | 8,0 |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakte aus Cajanus cajan (Beispiel 1-3) | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 11:**

| **Nachtcremerezepturen K8 bis K14** | | | | | | | | |
|---|---|---|---|---|---|---|---|---|
| (Alle Angaben in Gew.-% bez. auf das kosmetische Mitteln) | | | | | | | | |

| INCI Bezeichnung | K8 | K9 | K10 | K11 | K12 | K13 | K14 | V2 |
|---|---|---|---|---|---|---|---|---|
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt aus Cajanus cajan | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 12:**

| **W/O Bodylotion Rezepturen K15 bis K21** | | | | | | | | |
|---|---|---|---|---|---|---|---|---|
| (Alle Angaben in Gew.-% bez. auf das kosmetische Mitteln) | | | | | | | | |

| INCI-Bezeichnung | K15 | K16 | K17 | K18 | K19 | K20 | K21 | V3 |
|---|---|---|---|---|---|---|---|---|
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄*7H₂O | 1,0 | 1,0 | 1,0 | 1,0 | 1,0 | 1,0 | 1,0 | 1,0 |
| Extrakt aus Cajanus cajan | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 13:**

| Rezepturen für Coriditioner I Kosmetische Zubereitungen Conditioner (Wasser, Konservierungsmittel ad 100 Gew.-%) | | | | | | |
|---|---|---|---|---|---|---|
| **Zusammensetzung (INCI)** | **1 Gew.- %** | **2 Gew.- %** | **3 Gew.- %** | **4 Gew.- %** | **5 Gew: %** | **6 Gew.- %** |
| **Dehyquart® A** Cetrimonium Chloride | 4,0 | 4,0 | | | 3,0 | |
| **Dehyquart L® 80** Dicocoylmethylethoxymonium Methosulfate (and) Propylenglycol | | | 1,2 | 1,2 | | 1,0 |
| **Eumulgin® B2** Ceteareth-20 | 0,8 | | - | 0,8 | - | 1,0 |
| **Eumulgin® VL 75** Lauryl Glucoside (and) Polyglyceryl-2 Polyhydroxystearate (and) Glycerin | - | 2,0 | 2,0 | - | 0.8 | - |
| **Lanette® O** Cetearyl Alcohol | 3,0 | 3,0 | 3,0 | 3,0 | 3,0 | 3,0 |
| **Cutina® GMS** Glyceryl Stearate | - | 0,5 | - | 0,5 | - | 1,0 |
| **Lamesoft® PO 65** Coco-Glucosid (and) Glyceryl Oleate | | - | 3,0 | - | - | 3,0 |
| **Cetiol® J 600** Oleyl Erucate | - | 0,5 | - | 1,0 | - | 1,0 |
| **Eutanol® G** Octyldodecanol | - | - | 1,0 | - | - | 1,0 |
| **Nutrilan® Keratin W** Hydrolyzed Keratin | 5,0 | - | - | 2,0 | - | - |
| **Generol® 122 N** Soja Sterol | - | - | - | - | 1,0 | 1,0 |
| **Extrakt aus Cajanus cajan** | 1,0 | 1,0 | 1,0 | 1,0 | 1,0 | 1,0 |
| **Copherol® 1250** Tocopheryl Acetate | - | - | 0,1 | 0,1 | - | - |
| (1-4) Haarspülung, (5-6) Haarkur | | | | | | |

**Tabelle 13:**

| **Rezepturen für Conditioner II Kosmetische Zubereitungen Conditioner (Wasser, Konservierungsmittel ad 100 Gew.-%)** | | | | |
|---|---|---|---|---|
| **Zusammensetzung (INCI)** | **7 Gew.%** | **8 Gew.%** | **9 Gew.%** | **10 Gew.%** |
| **Texapon®NSO** Sodium Laureth Sulfate | 38,0 | 38,0 | 25,0 | - |
| **Texapon® SB 3** Disodium Laureth Sulfosuccinate | - | - | 10,0 | - |
| **Plantacare® 818** Coco Glucosides | 7,0 | 7,0 | 6,0 | - |
| **Plantacare® PS 10** Sodium Laureth Sulfate (and) Coco Glucosides | - | - | - | 20,0 |
| **Dehyton® PK 45** Cocamidopropyl Betaine | - | - | 10,0 | |
| **Lamesoft® PO 65** Coco-Glucosid (and) Glyceryl Oleate | 3,0 | | | 4,0 |
| **Lamesoft® LMG** Glyceryl Laurate (and) Potassium Cocoyl Hydrolyzed Collagen | - | 5,0 | - | |
| **Euperlan® PK 3000 AM** Glycol Distearate (and) Laureth4 (and) Cocamidopropyl Betaine | - | 3,0 | 5,0 | 5,0 |
| **Extrakt aus Cajanus cajan** | 1,0 | 1,0 | 1,0 | 1,0 |
| **Arlypon®** F Laureth-2 | 3,0 | 3,0 | 1,0 | - |
| **Sodium Chloride** | - | 1,5 | - | 1,5 |
| (7-8) Duschbad, (9) Duschgel, (10) Waschlotion | | | | |

**Tabelle 13:**

| **Kosmetische Zubereitungen Shampoo (Wasser, Konservierungsmittel ad 100 Gew.-%)** | | | | | | |
|---|---|---|---|---|---|---|
| **Zusammensetzung (INCI)** | **15** | **16** | **17** | **18** | **19** | **20** |
| **Texapon® NSO** Sodium Laureth Sulfate | 30,0 | | | 30,0 | 25,0 | |
| **Texapon® K 14 S** Sodium Myreth Sulfate | | 30,0 | | | | 30,0 |
| **Texapon® SB 3** Disodium Laureth Sulfosuccinate | | 10,0 | | | | |
| **Plantacare® 818** Coco Glucosides | 4,0 | | | | | |
| **Plantacare® 2000** Decyl Glucoside | | 4,0 | | | | |
| **Plantacare® PS 10** Sodium Laureth Sulfate (and) Coco Glucosides | | | 20,0 | | | |
| **Dehyton® PK 45** Cocamidopropyl Betaine | 5,0 | | | 10,0 | | 10,0 |
| **Gluadin® WK** Sodium Cocoyl Hydrolyzed Wheat Protein | | | | | 8,0 | |
| **Lamesoft® PO 65** Coco-Glucosid (and) Glyceryl Oleate | - | - | - | - | 2,0 | 2,0 |
| **Nutrilan® Keratin W** Hydrolyzed Keratin | 5,0 | - | - | - | | - |
| **Gluadin® W 40** Hydrolyzed Wheat Protein | - | 2,0 | - | 2,0 | - | - |
| **Euperlan® PK 3000 AM** Glycol Distearate (and) Laureth-4 (and) Cocamidopropyl Betaine | - | - | - | 3,0 | 3,0 | - |
| **Panthenol** | - | - | - | - | - | 0,2 |
| **Extrakte aus Cajanus cajan** | 1,0 | 1,0 | 1,0 | 1,0 | 1,0 | 1,0 |
| **Arlypon® F** Laureth-2 | 1,5 | - | - | - | - | - |
| **Sodium Chloride** | - | 1,6 | 2,0 | 2,2 | - | 3,0 |

**Tabelle 13:**

| **Kosmetische Zubereitungen Duschbad Two in One" (Wasser, Konservierungsmittel ad 100 Gew.-%)** | | | | |
|---|---|---|---|---|
| **Zusammensetzung (INCI)** | **11** | **12** | **13** | **14** |
| **Texapon® NSO** Sodium Laureth Sulfate | 30,0 | 25,0 | | 25,0 |
| **Plantacare® 818** Coco Glucosides | | | | 8,0 |
| **Plantacare® 2000** Decyl Glucoside | | 8,0 | | |
| **Plantacare® PS 10** Sodium Laureth Sulfate (and) Coco Glucosides | | | 20,0 | |
| **Dehyton® PK 45** Cocamidopropyl Betaine | | 10,0 | 10,0 | |
| **Lamesoft® PO 65** Coco-Glucosid (and) Glyceryl Oleate | 5,0 | | | |
| **Lamesoft® LMG** Glyceryl Laurate (and) Potassium Cocoyl Hydrolyzed Collagen | | 5,0 | 5,0 | |
| **Gluadin® WQ** Laurdimonium Hydroxapropyll Hydrolyzed Wheat Protein | 3,0 | | | |
| **Gluadin® WK** Sodium Cocoyl Hydrolyzed Wheat Protein | | | | |
| **Euperlan® PK 3000 AM** _Glycol Distearate (and) Laureth4 (and) Cocamidopropyl Betaine | 5,0 | 3,0 | 4,0 | - |
| **Panthenol** | 0,5 | - | - | 0,5 |
| **Extrakte aus Cajanus cajan** | 1,0 | 1,0 | 1,0 | 1,0 |
| **Arlypon® F** Laureth-2 | 2,6 | 1,6 | - | 1,0 |
| **Sodium Chloride** | - | - | - | - |

**Tabelle 13:**

| **Kosmetische Zubereitungen Schaumbad (Wasser, Konservierungsmittel ad 100 Gew.-%)** | | | | | |
|---|---|---|---|---|---|
| **Zusammensetzung** Bezeichnung nach INCI | 21 | 22 | 23 | 24 | 25 |
| **Texapon® NSO** Sodium Laureth Sulfate | - | 30,0 | 30,0 | - | 25,0 |
| **Plantacare® 818** Coco Glucosides | - | 10,0 | - | - | 20,0 |
| **Plantacare® PS 10** Sodium Laureth Sulfate (and) Coco Glucosides | 22,0 | - | 5,0 | 22,0 | - |
| **Dehyton® PK 45** Cocamidopropyl Betaine | 15,0 | 10,0 | 15,0 | 15,0 | 20,0 |
| **Monomuls® 90-O 18** Glyceryl Oleate | 0,5 | | | | |
| **Lamesoft® PO 65** Coco-Glucosid (and) Glyceryl Oleate | | 3,0 | | 3,0 | |
| **Cetiol® HE** PEG-7 Glyceryl Cocoate | | | 2,0 | | 2,0 |
| **Nutrilan® I-50** Hydrolyzed Collagen | 5,0 | | | | |
| **Gluadin® W 40** Hydrolyzed Wheat Gluten | | 5,0 | | 5,0 | |
| **Gluadin® WK** Sodium Cocoyl Hydrolyzed Wheat Protein | | | | 7,0 | |
| **Euperlan® PK 3000 AM** Glycol Distearate (and) Laureth-4 (and) Cocamidopropyl Betaine | 5,0 | - | - | 5,0 | - |
| **Arlypon® F** Laureth-2 | | | 1,0 | | |
| **Sodium Chloride** | 1,0 | | 1,0 | | 2,0 |
| **Extrakte aus Cajanus cajan** | 1,0 | 1,0 | 1,0 | 1,0 | 1,0 |

**Tabelle 13:**

| **Kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **31** | **32** | **33** | **34** | **35** | **36** | **37** | **38** | **39** | **40** |
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | - | - | 2,0 | - | 2,0 | 4,0 | 2,0 | 4,0 | 4,0 | 1,0 |
| **Antaron® V 216** PVP / Hexadecene Copolymer | - | - | - | - | - | 3,0 | - | - | - | 2,0 |
| **Myritol® 818** Cocoglycerides | 5,0 | - | 10,0 | - | 8,0 | 6,0 | 6,0 | - | 5,0 | 5,0 |
| **Finsolv® TN** C12/15 Alkyl Benzoate | - | 6,0 | - | 2,0 | - | - | 3,0 | - | - | 2,0 |
| **Cetiol® J 600** Oleyl Erucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol / Bisabolol** | 1,2 | 1,2 | 1,2 | 1,2 | 1,2 | 1,2 | 1,2 | 1,2 | 1,2 | 1,2 |
| **Extrakt aus Cajanus cajan** | 1,0 | 1,0 | 1,0 | 1,0 | 1,0 | 1,0 | 1,0 | 1,0 | 1,0 | 1,0 |
| **Copherol® F** 1300 Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylberizimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (31) W/O-Sonnenschutzcreme, (32-34) W/O-Sonnenschutzlotion, (35, 38, 40) O/W-Sonnenschutzlotion (36, 37, 39) O/W-Sonnenschutzcreme | | | | | | | | | | |
| Alle in der Tabelle 10-13 aufgeführten und verwendeten Substanzen mit registriertem Warenzeichen ® sind Marken und Produkte der COGNIS Gruppe. | | | | | | | | | | |

## Patentansprüche

1. Kosmetische und/oder pharmazeutische Zubereitungen, enthaltend einen Extrakt der Pflanze Cajanus cajan.

2. Zubereitungen nach Anspruch 1, **dadurch gekennzeichnet, dass** sie den Pflanzenextrakt in Mengen von 0,001 bis 25 Gew.-% berechnet als Trockengewicht bezogen auf die Gesamtmenge der Zubereitungen enthalten, mit der Maßgabe, dass sich die Mengenangaben mit Wasser und gegebenenfalls weiteren Hilfs- und Zusatzstoffen zu 100 Gew.-% addieren.

3. Zubereitungen nach Anspruch 1, **dadurch gekennzeichnet, dass** sie Substanzen enthalten, die ausgewählt sind aus der Gruppe, die gebildet wird von Tanninen, Phytosterolen, Proteinen, Kohlenhydraten, Phenolsäuren und Triterpene.

4. Verwendung von Extrakten der Pflanze Cajanus cajan in Pflegemitteln für die Haut und/oder Haare.

5. Verwendung von Extrakten der Pflanze Cajanus cajan in Pflegemitteln mit lindernder, wohltuender und irritationshemmender Wirkung, insbesondere für sensitive Haut.

6. Verwendung von Extrakten der Pflanze Cajanus cajan in Sonnenschutzmitteln.

7. Verwendung nach Anspruch 6 in Mittel gegen die Schädigung von menschlichen Hautzellen durch UV-Strahlung, insbesondere von Fibroblasten und/oder Keratinocyten durch UVA-Strahlung und/oder UVB-Strahlung.

8. Verwendung von Extrakten der Pflanze Cajanus cajan als anti-inflammatorische Addititve.

9. Verwendung von Extrakten der Pflanze Cajanus cajan als Antioxidantien bzw. Radikalfänger.

10. Verwendung von Extrakten der Pflanze Cajanus cajan als Mittel gegen Rosacea.

11. Verwendung von Extrakten der Pflanze Cajanus cajan als Mittel gegen hormonell und/oder bakteriell bedingte Hautveränderungen, insbesondere gegen Akne.

12. , Verwendung von Extrakten aus den Blättern der Pflanze Cajanus cajan in Pflegemitteln gegen die Hautalterung zur vorbeugenden oder heilenden Behandlung von Alterserscheinungen der Haut.

13. Verwendung von Extrakten aus den Blättern der Pflanze Cajanus cajan als Protease-inhibierendes Mittel, insbesondere als MMP-, Collagenase- und/oder Elastase-inhibierendes Mittel.

14. Verwendung von Extrakten aus den Blättern der Pflanze Cajanus cajan als Mittel mit einer Anti-Glykosylierungs-Aktivität, insbesondere gegen die Glykosylierung von kutanen Proteinen und bevorzugt gegen die Glykosylierung von Collagen;

15. Verwendung von Extrakten aus den Blättern der Pflanze Cajanus cajan in Haarpflegemitteln, insbesondere zur Verbesserung der Kämmbarkeit.

16. Verfahren zur Herstellung eines Extraktes der Pflanze Cajanus cajan, **dadurch gekennzeichnet, dass** zur Extraktion Lösungsmittel oder Mischungen dieser Lösungsmittel verwendet werden, welche ausgewählt sind aus der Gruppe, die gebildet wird von destilliertem oder nicht destilliertem Wasser, niedermolekularen Alkoholen, Estern Kohlenwasserstoffe, Ketone oder halogenhaltige Kohlenwasserstoffe und der so erhaltene Extrakt gegebenenfalls getrocknet wird.
